# FASCICULE DE BREVET EUROPEEN

(11) **EP 0 663 901 B1**
(45) Date de publication et mention de la délivrance du brevet: **19.03.1997**
(21) Numéro de dépôt: 93921981.2
(22) Date de dépôt: 04.10.1993
(51) Int. Cl.: C07C 271/22, C07D 305/14, C07C 271/12

(54) **PROCEDE DE PREPARATION STEREOSELECTIVE D'UN DERIVE DE LA BETA-PHENYLISOSERINE ET SON UTILISATION POUR LA PREPARATION DE DERIVES DU TAXANE**
STEREOSELEKTIVES VERFAHREN ZUR HERSTELLUNG EINES BETA-PHENYLISOSERIN-DERIVATES SOWIE SEINE VERWENDUNG ZUR HERSTELLUNG VON TAXAN-DERIVATEN
METHOD FOR THE STEREOSELECTIVE PREPARATION OF A DERIVATIVE OF BETA-PHENYLISOSERINE AND ITS USE IN THE PREPARATION OF TAXANE DERIVATIVES

(30) Priorité: 05.10.1992 FR 9211740
(43) Date de publication de la demande: 26.07.1995
(73) Titulaire: RHONE-POULENC RORER S.A., 92160 Antony (FR)
(72) Inventeur: DENIS, Jean-Noel, F-38410 Uriage (FR); GREENE, Andrew, Saint-Martin-d'Uriage, F-38410 Uriage (FR); KANAZAWA, Alice, F-38000 Grenoble (FR)
(74) Mandataire: Pilard, Jacques
(86) Numéro de dépôt international: FR9300966
(87) Numéro de publication internationale: WO9407847

(56) Documents cités:
- EP-A- 0 414 610
- WO-A-91/17976
- WO-A-91/17977

## Description

La présente invention concerne un procédé de préparation stéréosélective d'un dérivé de la β-phénylisosérine de formule générale: 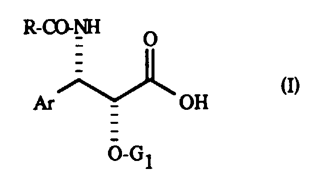 dans laquelle
Ar représente un radical aryle,
R représente un radical phényle ou α- ou β-naphtyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone, ou un radical R₁-O dans lequel R₁ représente :
   - un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 11 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
   - ou un radical phényle éventuellement substitué par un ou plusieurs atomes ou radicaux choisis parmi les atomes d'halogène et les radicaux alcoyles contenant 1 à 4 atomes de carbone, alcoyloxy contenant 1 à 4 atomes de carbone,
   - ou un radical hétérocyclique azoté saturé ou non saturé contenant 4 ou 6 chaînons et éventuellement substitué par un ou plusieurs radicaux alcoyles contenant 1 à 4 atomes de carbone,
   étant entendu que les radicaux cycloalcoyles, cycloalcényles ou bicycloalcoyles peuvent être éventuellement substitués par un ou plusieurs radicaux alcoyles contenant 1 à 4 atomes de carbone, et
G₁ représente un groupement protecteur de la fonction hydroxy choisi parmi les radicaux méthoxyméthyle, éthoxy-1 éthyle, benzyloxyméthyle, trichloro-2,2,2 éthoxyméthyle, tétrahydrofurannyle, tétrahydropyrannyle, β-(triméthylsilyl) éthoxyméthyle, trialcoylsilyle dont les radicaux alcoyles contiennent 1 à 4 atomes de carbone, ou -CH₂-Ph dans lequel Ph représente un radical phényle éventuellement substitué par un ou plusieurs atomes ou radicaux, identiques ou différents, choisis parmi les atomes d'halogène, les radicaux alcoyles contenant 1 à 4 atomes de carbone et les radicaux alcoxy contenant 1 à 4 atomes de carbone.

De préférence Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alkylamino, dialkylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone, que les radicaux alcényles et alcynyles contiennent 3 à 8 atomes de carbone et que les radicaux aryles sont des radicaux phényles ou *α-* ou β-naphtyles.

Plus particulièrement, Ar représente un radical phényle éventuellement substitué par un ou plusieurs atomes ou radicaux, identiques ou différents, choisis parmi les atomes d'halogène et les radicaux alcoyles, alcoxy, amino, alcoylamino, dialcoylamino, acylamino, alcoxycarbonylamino et trifluorométhyle.

Plus particulièrement encore, Ar représente un radical phényle éventuellement substitué par un atome de chlore ou de fluor, ou par un radical alcoyle (méthyle), alcoxy (méthoxy), dialcoylamino (diméthylamino), acylamino (acétylamino) ou alcoxycarbonylamino (tert-butoxycarbonylamino).

D'un intérêt encore plus particulier sont les produits de formule générale (I) dans laquelle Ar représente un radical phényle et R représente un radical phényle ou tert.butoxy et G₁ représente un radical benzyle ou p-méthoxybenzyle.

Les produits de formule générale (I), et en particulier ceux pour lesquels G₁ représente -CH₂-Ph qui sont des produits nouveaux constituant un autre objet de la présente invention, sont particulièrement utiles pour préparer le taxol ou le Taxotère et leurs analogues par condensation sur un dérivé de la baccatine III ou de la désacétyl-10 baccatine III dont les fonctions hydroxy sont convenablement protégées en opérant dans les conditions qui sont décrites par exemple dans les brevets européens EP 0 336 840 ou EP 0 336 841.

Il est connu de préparer des analogues du produit de formule générale (I) à partir d'un acide β-phénylglycidique en opérant, par exemple, dans les conditions décrites dans le brevet européen EP 0 414 610.

Il a maintenant été trouvé que les produits de formule générale (I) peuvent être obtenus directement, avec une très bonne énantio- et dia-stéréosélectivité, par la mise en oeuvre d'un procédé qui nécessite la réalisation d'un nombre d'étapes beaucoup plus faibles que selon les procédés antérieurement connus.

Selon la présente invention, les produits de formule générale (I) peuvent être obtenus par action d'une N-carbonylarylimine de formule générale :

Ar-CH=N-CO-R (II)

dans laquelle Ar et R sont définis comme précédemment, sur l'anion d'un amide optiquement actif d'un acide hydroxyacétique protégé de formule générale : 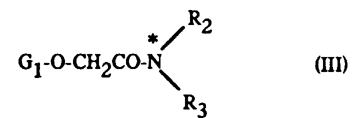 dans laquelle G₁ est défini comme précédemment et 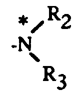 représente le reste d'une base organique optiquement active, suivie de l'hydrolyse du produit ainsi obtenu de formule générale : 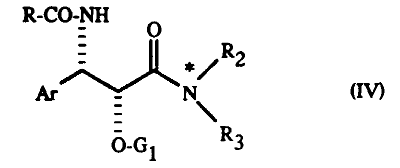 dans laquelle R, Ar, G₁ et 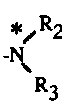 sont définis comme précédemment.

Il est particulièrement avantageux d'utiliser un amide de formule générale (III) dans laquelle 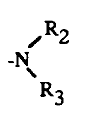 représente un reste L(+)- 2,10-camphorsultame de formule :

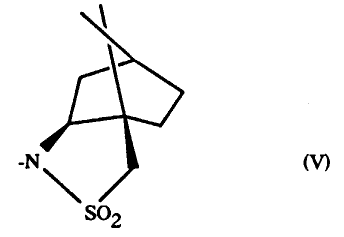

Le procédé selon l'invention est généralement mis en oeuvre en faisant réagir la N-carbonylarylimine de formule générale (II), éventuellement préparée in situ, sur l'amide de l'acide hydroxyacétique protégé préalablement anionisé. L'anionisation s'effectue généralement au moyen d'un amidure de métal alcalin. Parmi les amidures appropriés peuvent être cités le bis(triméthylsilyl) amidure de sodium (NHMDS), de lithium (LHMDS) ou de potassium (KHMDS), le diisopropylamidure de lithium (LDA), le diéthylamidure de lithium (LDEA), le dicyclohexylamidure de lithium (LDCHA), (CH₃)₃SiN(R')Li (R' = alcoyle, cycloalcoyle, aryle) et tBuLi. D'un intérêt tout particulier est le bis(triméthylsilyl) amidure de lithium qui permet d'obtenir un rendement élevé et une excellente stéréosélectivité.

Généralement l'anionisation est effectuée dans un solvant organique inerte tel qu'un éther comme le tétrahydrofuranne à une température inférieure à 0°C et de préférence voisine de -78°C.

L'action du produit de formule générale (II) sur le produit de formule générale (III) préalablement anionisé s'effectue généralement dans le même solvant et à la même température.

Le produit de formule générale (IV) est hydrolysé en produit de formule générale (I) au moyen d'une base minérale telle que la soude, la potasse ou la lithine en milieu aqueux ou hydro-organique. Il est particulièrement avantageux d'opérer dans un mélange tétrahydrofuranne-eau en présence d'eau oxygénée. La température de la réaction est généralement comprise entre -10 et 20°C et de préférence au voisinage de 0°C.

La N-carbonylarylimine de formule générale (II) dans laquelle Ar est défini comme précédemment et R représente un radical t.butoxy est un produit nouveau qui constitue un autre objet de la présente invention.

La N-carbonylarylimine de formule générale (II) peut être obtenue par action d'un halogénure de benzoyle éventuellement substitué ou d'un dérivé réactif de formule générale :

R₁-O-CO-X (VI)

dans laquelle R₁ est défini comme précédemment et X représente un atome d'halogène (fluor, chlore) ou un reste -O-R₁ ou -O-CO-OR₁, sur un produit de formule générale:

Ar-CH=N-Z (VII)

dans laquelle Ar est défini comme précédemment et Z représente un groupement réactif tel qu'un radical trialkylsilyle comme le radical triméthylsilyle.

Généralement l'action de l'halogénure de benzoyle éventuellement substitué ou du produit de formule générale (VI) sur le produit de formule générale (VII) est effectuée par chauffage dans un solvant organique tel qu'un ester comme l'acétate d'éthyle ou un hydrocarbure aliphatique halogéné comme le dichlorométhane ou le chloroforme ou un hydrocarbure aromatique comme le toluène ou le benzène.

L'imine de formule générale (VII) peut être obtenue à partir de l'aldéhyde de formule générale :

Ar-CHO (VIII)

dans laquelle Ar est défini comme précédemment selon les méthodes connues. Par exemple, le produit de formule générale (VII) dans laquelle Z représente un radical triméthylsilyle peut être obtenu selon DJ. Hart et coll., J. Org. Chem., 48, 289 (1983) par action du bis(triméthyldisilyl) amidure de lithium (LHMDS), éventuellement préparé in situ par action du butyllithium sur la bis-(triméthylsilylamine), sur l'aldéhyde correspondant de formule générale (VIII).

La N-carbonylarylimine de formule générale (II) peut aussi être préparée in situ par action d'une base forte telle qu'un amidure comme le bis-(triméthylsilyl) amidure de lithium, sur un thioéther de formule générale : 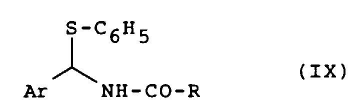 dans laquelle Ar et R sont définis comme précédemment.

L'amide optiquement active de formule générale (III) peut être obtenue par action d'un dérivé activé d'un acide hydroxyacétique protégé de formule générale :

G₁-O-CH₂-COOH (X)

dans laquelle G₁ est défini comme précédemment, tel que l'halogénure ou l'anhydride sur la base chirale correspondante éventuellement anionisée.

Le produit de formule générale (I) peut être utilisé pour préparer les dérivés du taxane thérapeutiquement actifs de formule générale : 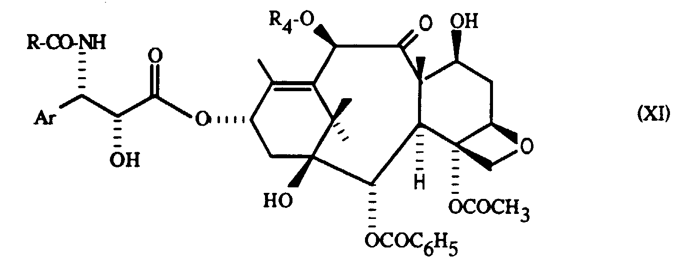 dans laquelle Ar et R sont définis comme précédemment et R₄ représente un atome d'hydrogène ou un radical acétyle dans un procédé qui consiste à faire réagir un produit de formule générale (I) sur un dérivé de la baccatine III ou de la désacétyl-10 baccatine III de formule générale : 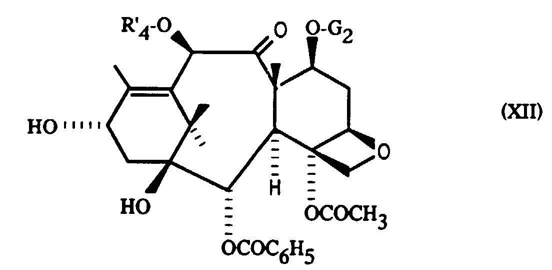 dans laquelle G₂ représente un groupement protecteur de la fonction hydroxy tel qu'un radical trichloro-2,2,2 éthoxycarbonyle ou trialcoylsilyle et R'₄ représente un radical acétyle ou un groupement protecteur de la fonction hydroxy tel qu'un radical trichloro-2,2,2 éthoxycarbonyle, pour obtenir un produit de formule générale : 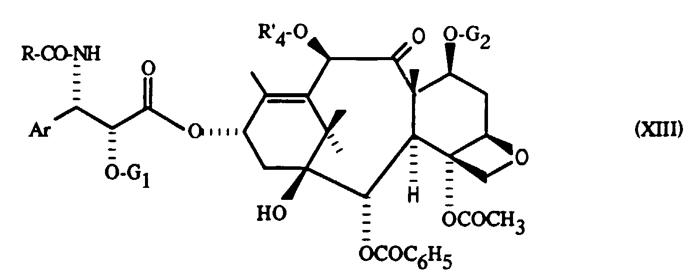 dans laquelle R, Ar, G₁, G₂ et R'₄ sont définis comme précédemment, dont les groupements protecteurs G₁, G₂ et éventuellement R'₄ sont remplacés par des atomes d'hydrogène simultanément ou successivement.

Généralement, l'estérification d'un produit de formule générale (XII) par un produit de formule générale (I) est effectuée en présence d'un agent de condensation tel qu'un carbodiimide comme le dicyclohexylcarbodiimide ou un carbonate réactif comme le pyridyl-2 carbonate et d'un agent d'activation tel qu'une aminopyridine comme la diméthylamino-4 pyridine ou la pyrrolidino-4 pyridine en opérant dans un solvant organique tel qu'un hydrocarbure aromatique (benzène, toluène, xylène, éthylbenzène, isopropylbenzène, chlorobenzène), un éther (tétrahydrofuranne), un nitrile (acétonitrile) ou un ester (acétate d'éthyle) à une température comprise entre 0 et 90°C.

Lorsque G₁ représente un radical méthoxyméthyle, éthoxy-1 éthyle, benzyloxyméthyle, trichloro-2,2,2 éthoxyméthyle, tétrahydrofurannyle, tétrahydropyrannyle, β-triméthylsilyléthoxyméthyle ou trialcoylsilyle dont les radicaux alcoyles contiennent 1 à 4 atomes de carbone, le remplacement des groupements protecteurs G₁, G₂ et éventuellement R'₄ du produit de formule générale (XIII) est effectué soit par le zinc, éventuellement associé au cuivre, en présence d'acide acétique ou au moyen d'un acide minéral ou organique tel que l'acide chlorhydrique ou l'acide acétique éventuellement en solution dans un alcool aliphatique contenant 1 à 3 atomes de carbone, en présence de zinc éventuellement associé à du cuivre, lorsque l'un des groupements protecteurs représente un radical trichloro-2,2,2 éthoxycarbonyle soit par traitement par un acide minéral ou organique tel que l'acide chlorhydrique ou l'acide acétique éventuellement en solution dans un alcool aliphatique contenant 1 à 3 atomes de carbone lorsque l'un des groupements protecteurs représente un radical silylé.

Lorsque G₁ représente un radical -CH₂-Ph ou éventuellement benzyloxyméthyle, on effectue d'abord le remplacement des groupements protecteurs G₂ et éventuellement R'₄ par des atomes d'hydrogène dans les conditions décrites ci-dessus pour obtenir le produit de formule générale : 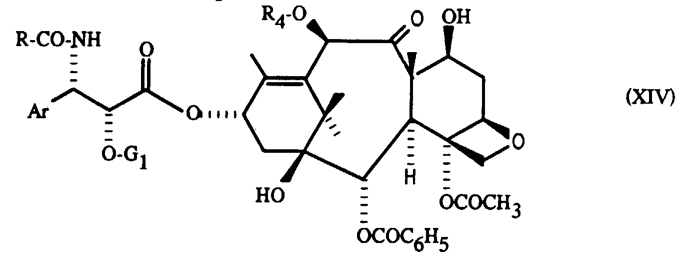 dans laquelle R, Ar et R₄ sont définis comme précédemment dont le groupement Ph-CH₂- ou éventuellement benzyloxyméthyle est remplacé par un atome d'hydrogène pour obtenir le produit de formule générale (XI).

Le remplacement du groupement Ph-CH₂- ou éventuellement benzyloxyméthyle du produit de formule générale (XIV) par un atome d'hydrogène s'effectue généralement par hydrogénolyse au moyen d'hydrogène en présence d'un catalyseur tel que le palladium noir en opérant dans un solvant organique tel que l'acide acétique à une température comprise entre 0 et 60°C, de préférence voisine de 40°C. Il peut être avantageux d'opérer sous pression et éventuellement en présence d'une quatité catalytique d'un acide tel que l'acide perchlorique. Le même remplacement s'effectue aussi par l'action de la dichlorodicyanobenzoquinone (DDQ) dans un solvant organique tel que le dichlorométhane ou l'acétonitrile.

Les dérivés du taxane de formule générale (XI) ainsi obtenus peuvent être éventuellement purifiés par application des techniques habituelles.

Les exemples suivants illustrent la présente invention.

### EXEMPLE 1

Dans un ballon monocol de 10 cm3 muni d'un système d'agitation magnétique, on introduit, sous atmosphère d'argon, 287 mg (0,79 mmole) de L-N-(benzyloxyacétyl) 2,10-camphorsultame et 3 cm3 de tétrahydrofuranne anhydre. On refroidit la solution à -78°C puis on ajoute, goutte à goutte, 0,8 cm3 (0,8 mmole) d'une solution 1M de bis(triméthylsilyl)amidure de lithium dans le tétrahydrofuranne. On laisse réagir pendant 1 heure à -78°C puis on ajoute 248 mg (1,21 mmole) de N-t.butoxycarbonylbenzylimine en solution dans 1,7 cm3 de tétrahydrofuranne anhydre. Après 15 minutes de réaction à -78°C, on hydrolyse le mélange réactionnel par addition d'une solution aqueuse saturée de chlorure d'ammonium. On extrait 2 fois par du dichlorométhane. Les phases organiques réunies sont lavées 2 fois avec de l'eau puis 1 fois avec une solution aqueuse saturée de chlorure de sodium puis séchées sur sulfate de magnésium anhydre. Après filtration et élimination des solvants sous pression réduite, on obtient un résidu (578 mg) qui est purifié par chromatographie sur gel de silice en éluant avec un mélange hexane-acétate d'éthyle (85-15 en volumes). On obtient ainsi, avec un rendement de 66 %, 294 mg (0,52 mmole) de L(+)-N-[benzyloxy-2 t-butoxycarbonylamino-3 phényl-3 propionyl] 2,10-camphorsultame-syn dont les caractéristiques sont les suivantes :
- point de fusion : 79°C puis 130°C (dichlorométhane-hexane)
- pouvoir rotatoire : [α]²⁵_{D} = +53° (c = 0,98 ; chloroforme)
- spectre infra-rouge (film) : principales bandes d'absorption caractéristiques à 3450, 3050, 3020, 2975, 1720, 1500, 1460, 1420, 1395, 1370, 1340, 1280, 1240, 1220, 1170, 1140, 1100, 1070, 1020, 860, 810, 760, 750 et 700 cm⁻¹.
- spectre de RMN du proton (300 MHz ; CDCl₃ ; déplacements chimiques en ppm ; constantes de couplage J en Hz) ;
   0,99 (s, 3H) ;1,1-1,6 (m, 2H) ; 1,28 (s, 3H) ; 1,39 (s, 9H) ; 1,83-2,25 (m, 5H) ; 3,51 (AB_{q}, J_{AB} = 13,7, δ_{A}-δ_{B} = 21,4, 2H) ; 3,94-4,03 (m, 1H); 4,36 (AB_{q}, J_{AB} = 11,4, δ_{A}-δ_{B} = 120, 2H) ; 4,86 (s large, 1H); 5,33 (d, J = 9,8, 1H); 5,60 (d, J = 9,8, 1H); 6,9-7,05 (m, 2H) ; 7,14-7,4 (m, 8H).
- spectre de RMN du ¹³C (75,47 MHz ; CDCl₃):
   19,97 (CH₃) ; 20,64 (CH₃) ; 26,59 (CH₂) ; 28,24 (CH₃) ; 32,81 (CH₂) ; 37,53 (CH₂) ; 44,49 (CH) ; 47,92 (C) ; 48,89 (C) ; 53,11 (CH₂) ; 55,70 (CH) ; 65,07 (CH) ; 72,47 (CH₂) ; 79,32 (C) ; 81,29 (CH) ; 126,78 (CH) ; 127,17 (CH) ; 127,65 (CH) ; 127,84 (CH) ; 128,09 (CH) ; 136,72 (C) ; 139,54 (C) ; 154,95 (C) ; 169,90 (C).

Dans un ballon monocol de 10 cm3 muni d'un système d'agitation magnétique, on introduit, sous atmosphère d'argon, 66 mg (0,116 mmole) du produit obtenu précédemment et 1 cm3 d'un mélange tétrahydrofuranne-eau (4-1 en volumes). On refroidit à 0°C puis on ajoute 95 µl (0,93 mmole) d'eau oxygénée à 30 % en volumes et 20 mg (0,48 mmole) de lithine hydratée (LiOH, H₂O). On laisse réagir pendant 1 heure à 0°C puis on agite pendant 15 heures à 20°C. On ajoute alors une solution de 117 mg (0,93 mmole) de sulfite de sodium dans 0,7 cm3 d'eau. Après évaporation du tétrahydrofuranne, on ajoute de l'eau puis on extrait 3 fois la solution aqueuse basique obtenue par du dichlorométhane. La phase aqueuse basique est acidifiée à pH = 1-2 par addition d'une solution aqueuse d'acide chlorhydrique 2M et est extraite 6 fois par l'acétate d'éthyle. Les phases organiques réunies sont lavées par une solution aqueuse saturée de chlorure de sodium puis séchées sur sulfate de magnésium anhydre. Après filtration et élimination du solvant sous pression réduite, on obtient, avec un rendement de 70 %, 30 mg (0,081 mmole) d'acide benzyloxy-2 t.butoxycarbonylamino-3 phényl-3 propionique-2R,3S dont les caractéristiques sont les suivantes :
- spectre infra-rouge (film) : bandes d'absorption caractéristiques à 3700-2300, 3450, 3300, 3075, 3050, 3025, 2975, 2925, 1720, 1660, 1510, 1500, 1450, 1390, 1370, 1250, 1165, 1110, 1020, 860, 740 et 695 cm⁻¹.
- spectre de RMN du proton (200 MHz ; CDCl₃ ; déplacements chimiques en ppm ; constantes de couplage J en Hz) :
1,42 (s, 9H) ; 4,20 (s large, 1H); 4,52 (AB_{q}, J_{AB} = 11,6, δ_{A}-δ_{B} = 65, 2H) ; 5,30 (d déformé, J = 9,9, 1H); 5,78 (d déformé, J = 9,4, 1H); 6,2 (s large, 1H); 7,0-7,06 (m, 2H) ; 7,06-7,44 (m, 8H).
- spectre de RMN du ¹³C (50,3 MHz, CDCl₃):
28,24 (CH₃) ; 55,67 (CH) ; 72,90 (CH₂) ; 79,84 (CH) ; 80,49 (C) ; 126,60 (CH) ; 127,50 (CH) ; 127,95 (CH) ; 128,30 (CH) ; 136,40 (C) ; 139,36 (C) ; 155,66 (C) ; 173,08 (C).
-

| analyse élémentaire (C₂₁H₂₅O₅N) | | | |
|---|---|---|---|
| calculé | C% 67,91 | H% 6,78 | N% 3,77 |
| trouvé | 67,67 | 6,68 | 3,87 |

La N-(t.butoxycarbonyl)benzylimine peut être préparée de la manière suivante :

Dans un ballon monocol de 100 cm3 muni d'un système d'agitation magnétique, on introduit, sous atmosphère d'argon, 20 cm3 (95 mmoles) de bis-(triméthylsilyl) amine fraîchement distillée puis on refroidit à 0°C. On ajoute ensuite, goutte à goutte, 34 cm3 (85 mmoles) d'une solution de n-butyllithium 2,5M dans l'hexane. On laisse la température remonter au voisinage de 20°C puis on laisse réagir pendant 10 minutes. On refroidit à 0°C puis on ajoute 8,63 cm3 (85 mmoles) de benzaldéhyde fraîchement distillé. On laisse réagir à 0°C pendant 3 heures 30 minutes. Après élimination du solvant sous pression réduite, le résidu est distillé sous pression réduite. On obtient ainsi, avec un rendement de 92 %, 13,8 g (78 mmoles) de N-(triméthylsilyl)benzylimine dont les caractéristiques sont les suivantes :
- spectre infra-rouge (film) : principales bandes d'absorption caractéristiques à 3050, 3020, 2950, 2900, 2800, 2700, 1650, 1600, 1580, 1450, 1300, 1250, 1210, 1160, 1070, 1020, 970, 860, 840, 750 et 690 cm⁻¹.
- spectre de RMN du proton (200 MHz ; CDCl₃) : 0,3 (s, 9H) ; 7,42-7,56 (m, 3H) ; 7,77-7,90 (m, 2H) ; 9,02 (s, 1H).

Dans un ballon monocol de 100 cm3 muni d'un système d'agitation magnétique, on introduit, sous atmosphère d'argon, 2,92 g (16,5 mmoles) de l'imine obtenue précédemment puis 50 cm3 de chloroforme anhydre. On refroidit à 0°C puis on ajoute, goutte à goutte, 6,93 g (31,8 mmoles) de dicarbonate de di-t.butyle pur. Le mélange réactionnel est chauffé au reflux pendant 12 heures.

Après élimination du chloroforme sous pression réduite, le résidu est distillé sous pression réduite (1,3 Pa) à 103-105°C. On obtient ainsi, avec un rendement de 56 %, 1,91 g (9,3 mmoles) de N-(t.butoxycarbonyl)benzylimine dont les caractéristiques sont les suivantes :
- spectre infra-rouge (film) : 3050, 2970, 2925, 1730, 1650, 1605, 1590, 1485, 1460, 1320, 1275, 1260, 1220, 1155, 1000, 980, 885, 850, 755, 690 cm⁻¹.
- spectre de RMN du proton (200 MHz ; CDCl₃) 1,61 (s, 9H) ; 7,44-7,60 (m, 3H) ; 7,9-8,0 (m, 2H); 8,9 (s, 1H).

Le L-N-(benzyloxyacétyl) 2,10-camphorsultame peut être préparé de la manière suivante :

Dans un ballon monocol de 10 cm3 muni d'un système d'agitation magnétique, on introduit, sous atmosphère d'argon, 181 mg (0,84 mmole) de L(+)-bornanesultame-10,2 en solution dans 2 cm3 de toluène anhydre. On refroidit à 0°C puis on ajoute 50 mg (1,25 mmole) d'hydrure de sodium 60 % en dispersion dans l'huile minérale. On laisse réagir pendant 30 minutes à 0°C puis on ajoute 0,17 cm3 (1,08 mmole) de chlorure de benzyloxyacétyle. On laisse la température remonter à 20°C puis on laisse réagir pendant 2 heures. Le mélange réactionnel est dilué par addition de dichlorométhane puis on ajoute lentement de l'eau. La phase organique, séparée par décantation, est lavée avec de l'eau puis avec une solution aqueuse saturée de chlorure de sodium et enfin séchée sur sulfate de magnésium anhydre. Après filtration et élimination des solvants sous pression réduite, on obtient 511 mg d'un résidu huileux qui est purifié par chromatographie sur une colonne de gel de silice en éluant avec un mélange hexane-acétate d'éthyle (80-20 en volumes). On obtient ainsi, avec un rendement de 97 %, 294 mg (0,81 mmole) de L-N-(benzyloxyacétyl) 2,10-camphorsultame dont les caractéristiques sont les suivantes :
- spectre infra-rouge (film) : principales bandes d'absorption caractéristiques à 2980, 2970, 1710, 1460, 1420, 1395, 1340, 1270, 1245, 1225, 1170, 1140, 1115, 1065, 1040, 1030, 985, 950, 870, 800, 780, 750 et 700 cm⁻¹.
- spectre de RMN du proton (200 MHz ; CDCl₃) : 0,96 (s, 3H) ; 1,13 (s, 3H) ; 1,2-1,6 (m, 2H) ; 1,6-2,3 (m, 5H) ; 3,3-3,6 (m, 2H) ; 3,8-4,0 (m, 1H); 4,4-4,75 (m, 4H) ; 7,1-7,5 (m, 5H).

### EXEMPLE 2

Dans un ballon monocol de 5 cm3 muni d'un système d'agitation magnétique, on introduit, sous atmosphère d'argon, 42 mg (0,115 mmole) de L-N-(benzyloxyacétyl) 2,10-camphorsultame et 0,4 cm3 de tétrahydrofuranne anhydre. On refroidit à -78°C puis on ajoute 115 µl (0,115 mmole) d'une solution 1M de bis(triméthylsilyl) amidure de lithium dans le tétrahydrofuranne. On laisse réagir pendant 1 heure à -78°C puis on ajoute 72 mg (0,23 mmole) de N-t.butoxycarbonyl α-phénylthio benzylamine et 230 µl (0,23 mmole) d'une solution 1M de bis(triméthylsilyl)amidure de lithium dans le tétrahydrofuranne. On laisse réagir pendant 1 heure 30 minutes à -78°C puis on hydrolyse le mélange réactionnel par addition d'une solution aqueuse saturée de chlorure d'ammonium. On laisse la température remonter à 20°C puis on extrait 3 fois avec de l'éther. Les phases organiques réunies sont lavées 2 fois avec de l'eau puis 1 fois avec une solution aqueuse saturée de chlorure de sodium puis séchées sur sulfate de magnésium anhydre. Après filtration et élimination des solvants sous pression réduite, le résidu obtenu (114 mg) est purifié par chromatographie sur une colonne de gel de silice en éluant avec un mélange hexane-acétate d'éthyle (85-15 en volumes).

On obtient ainsi, avec un rendement de 54 %, 35 mg (0,062 mmole) de L(+)-N-(bmzyloxy-2 t.butoxycarbonylamino-3 phényl-3 propionyl) 2,10-camphorsultame-syn dont les caractéristiques sont identiques à celles du produit obtenu à l'exemple 1.

### EXEMPLE 3

Dans un ballon monocol de 10 cm3 muni d'un système d'agitation magnétique, on introduit, sous atmosphère d'argon, 94 mg (0,253 mmole) d'acide benzyloxy-2 t.butoxycarbonylamino-3 phényl-3 propionique-2R,3S en solution dans 3,5 cm3 de toluène anhydre. On ajoute ensuite 52,3 mg (0,253 mmole) de dicyclohexylcarbodiimide distillé. On laisse réagir pendant 5 minutes à une température voisine de 20°C puis on ajoute en une seule fois un mélange de 7,7 mg (0,063 mmole) de N,N-diméthylamino-4 pyridine et 56,3 mg (0,063 mmole) d'acétoxy-4 benzoyloxy-2α époxy-5β,20 dihydroxy-1,13α oxo-9 bis-(trichloro-2,2,2 éthoxycarbonyloxy)-7β,10β taxène-11. On laisse réagir pendant 20 heures à une température voisine de 20°C. On dilue le mélange réactionnel par addition de 40 cm3 d'acétate d'éthyle. La phase organique est lavée 1 fois avec 5 cm3 d'eau distillée, 2 fois avec 5 cm3 d'une solution aqueuse saturée d'hydrogénocarbonate de sodium puis 1 fois avec 5 cm3 d'une solution aqueuse saturée de chlorure de sodium et elle est enfin séchée sur sulfate de sodium anhydre. Après filtration et élimination des solvants sous pression réduite, on obtient un résidu (166 mg) qui est purifié par chromatographie sur une colonne de gel de silice en éluant avec un mélange éther-dichlorométhane (1-99 en volumes). On obtient ainsi, avec un rendement de 93 %, 73mg (0,0585 mmole) de t.butoxycarbonylamino-3 phényl-3 benzyloxy-2 propionate-(2R,3S) d'acétoxy-4 benzoyloxy-2α époxy-5β,20 hydroxy-1 oxo-9 bis-(trichloro-2,2,2 éthoxycarbonyloxy)-7β,10β taxène-11 yle-13α dont les caractéristiques sont les suivantes :
- pouvoir rotatoire (produit repurifié) : [α]²⁵_{D} = -32° (c = 0,86 ; chloroforme).
- spectre infra-rouge (film) : principales bandes d'absorption caractéristiques à 3450, 3050, 2970, 2920, 2900, 1760, 1740, 1720, 1600, 1580, 1490, 1450, 1375, 1242, 1175, 1165, 1100, 1060, 1000, 975, 960, 820, 770, 720 et 700 cm⁻¹.
- spectre de RMN du proton (200 MHz ; CDCl₃ ; déplacements chimiques en ppm ; constantes de couplage J en Hz) :
1,21 (s, 3H) ;1,30 (s, 3H) ; 1,35 (s, 9H) ; 1,8-2 (m, 1H); 1,86 (s, 3H) ; 2,01 (s, 3H) ; 2-2,2 (m, 2H) ; 2,26 (s, 3H) ; 2,57-2,68 (m, 1H); 3,91 (d, J = 7, 1H); 4,24 (s, 1H); 4,25 (AB_{q}, J_{AB} = 8,7, δ_{A}-δ_{B} = 43,8, 2H) ; 4,50 (AB_{q}, J_{AB} = 12, δ_{A}-δ_{B} = 109, 2H) ; 4,76 (AB_{q}, J_{AB} = 11,8, δ_{A}-δ_{B} = 91, 2H) ; 4,78 (AB_{q}, J_{AB} = 12, δ_{A}-δ_{B} = 7,6, 2H) ; 4,95 (d déformé, J = 10,5, 1H); 5,14-5,36 (m, 1H); 5,4-5,6 (m, 1H); 5,57 (q, J = 7,2 et 10,7, 1H); 5,71 (d, J = 7, 1H); 6,2-6,33 (m, 1H); 6,26 (s, 1H); 7-7,1 (m, 2H aromatiques) ; 7,22-7,86 (m, 11H aromatiques) ; 8,06-8,11 (m, 2H aromatiques).
-

| analyse élémentaire (C₅₆H₆₁O₁₈NCl₆) | | | |
|---|---|---|---|
| calculé | C% 53,86 | H% 4,92 | N% 1,12 |
| trouvé | 53,75 | 5,15 | 1,32 |

Dans un ballon monocol de 10 cm3 muni d'un système d'agitation magnétique, on introduit, sous atmosphère d'argon, 58 mg (0,0465 mmole) de l'ester obtenu précédemment en solution dans 3 cm3 d'acide acétique glacial. On ajoute ensuite 3 cm3 de méthanol puis 260 mg du couple zinc-cuivre (préparé à partir de 20g de zinc et 3 g de sulfate de cuivre monohydrate). On chauffe le milieu hétérogène noir à 65°C pendant 30 minutes. Après refroidissement à une température voisine de 20°C, on dilue le mélange réactionnel dans 40 cm3 d'acétate d'éthyle. On filtre sur célite puis lave les solides 3 fois avec 20 cm3 d'acétate d'éthyle. On élimine les solvants sous pression réduite. Le résidu obtenu est purifié par chromatographie préparative sur couche mince de gel de silice en éluant avec un mélange méthanol-dichlorométhane (5-95 en volumes). On obtient, avec un rendement de 91 %, 38 mg de t.butoxycarbonylamino-3 phényl-3 benzyloxy-2 propionate-(2R,3S) d'acétoxy-4 benzoyloxy-2α époxy-5β,20 trihydroxy-1,7β,10,β oxo-9 taxène-11 yle-13α dont les caractéristiques sont les suivantes :
- spectre infra-rouge (film) : bandes d'absorption caractéristiques à 3430, 3050, 2975, 2910, 2880, 1740, 1725, 1710, 1495, 1450, 1390, 1370, 1350, 1270, 1240, 1160, 1105, 1065 et 980 cm⁻¹.
- spectre de RMN du proton (200 MHz ; CDCl₃ ; déplacements chimiques en ppm ; constantes de couplage J en Hz) :
1,14 (s, 3H) ; 1,26 (s, 3H) 1,33 (s, 9H) ; 1,75 (s, 3H) ; 1,91 (s, 3H) ; 1,8-2,3 (m, 3H) ; 2,24 (s, 3H) ; 2,46-2,73 (m, 1H); 3,91 (d, J = 7, 1H); 4,12-4,38 (m, 3H) ; 4,20 (s, 1H); 4,51 (AB_{q}, J_{AB} = 12, δ_{A}-δ_{B} = 71, 2H) ; 4,94 (d, J = 7,5, 1H); 5,21 (s, 1H); 5,13-5,29 (m, 1H); 5,44-5,6 (m, 1H); 5,69 (d, J = 7, 1H); 6,27 (t déformé, J = 7,3 et 8,8, 1H); 7-7,1 (m, 2H aromatiques) ; 7,19-7,66 (m, 11H aromatiques) ; 8,08-8,12 (m, 2H aromatiques).
-

| analyse élémentaire (C₅₀H₅₉O₁₄N) | | | |
|---|---|---|---|
| calculé | C% 66,87 | H% 6,62 | N% 1,56 |
| trouvé | 66,65 | 6,72 | 1,73 |

Dans un ballon monocol de 5 cm3 muni d'un système d'agitation magnétique, on introduit, sous atmosphère d'argon, 14 mg (0,0156 mmole) du produit obtenu précédemment en solution dans 1,6 cm3 d'acide acétique glacial. On ajoute ensuite 5 mg de palladium noir puis on place le mélange sous atmosphère d'hydrogène. On chauffe et agite à 40°C puis laisse réagir pendant 6 heures. Après refroidissement à une température voisine de 20°C, le mélange réactionnel est dilué dans 5 cm3 d'acétate d'éthyle. Après filtration sur célite, on lave les solides avec 5 fois 5 cm3 d'acétate d'éthyle. Les phases organiques réunies sont lavées 3 fois avec 5 cm3 d'une solution aqueuse saturée d'hydrogénocarbonate de sodium, 3 fois avec 5 cm3 d'eau et 1 fois avec 5 cm3 d'une solution aqueuse saturée de chlorure de sodium puis elles sont séchées sur sulfate de sodium anhydre. Après filtration et élimination des solvants sous pression réduite, le résidu obtenu (14 mg) est purifié par chromatographie préparative sur couche mince de silice en éluant avec un mélange méthanol-dichlorométhane (5-95 en volumes). On obtient ainsi, avec un rendement de 67 %, 8,5 mg (0,0105 mmole) de t.butoxycarbonylamino-3 phényl-3 hydroxy-2 propionate-(2R,3S) d'acétoxy-4 benzoyloxy-2α époxy-5β,20 trihydroxy1,7β,10β oxo-9 taxène-11 yle-13α (ou Taxotère) dont les caractéristiques sont identiques à celles décrites dans la littérature.

## Revendications

1. Procédé de préparation stéréosélective d'un dérivé de la β-phénylisosérine de formule générale : 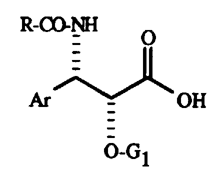 éventuellement sous forme de sel ou d'ester, dans laquelle
Ar représente un radical aryle,
R représente un radical phényle ou α- ou β-naphtyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone, ou un radical R₁-O dans lequel R₁ représente :
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 11 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les atomes d'halogène et les radicaux alcoyles contenant 1 à 4 atomes de carbone, alcoyloxy contenant 1 à 4 atomes de carbone,
- ou un radical hétérocyclique azoté saturé ou non saturé contenant 4 ou 6 chaînons et éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone, et
G₁ représente un groupement protecteur de la fonction hydroxy choisi parmi les radicaux méthoxyméthyle, éthoxy-1 éthyle, benzyloxyméthyle, trichloro-2,2,2 éthoxyméthyle, tétrahydrofurannyle, tétrahydropyrannyle, β-(triméthylsilyl) éthoxyméthyle, trialcoylsilyle dont les radicaux alcoyles contiennent 1 à 4 atomes de carbone, ou -CH₂-Ph dans lequel Ph représente un radical phényle éventuellement substitué par un ou plusieurs atomes ou radicaux, identiques ou différents, choisis parmi les atomes d'halogène, les radicaux alcoyles contenant 1 à 4 atomes de carbone et les radicaux alcoxy contenant 1 à 4 atomes de carbone,
caractérisé en ce que l'on fait réagir une N-carbonylarylimine de formule générale :
Ar-CH=N-CO-R
dans laquelle Ar et R sont définis comme précédemment sur un amide optiquement actif d'un acide hydroxyacétique protégé, préalablement anionisé, de formule générale: 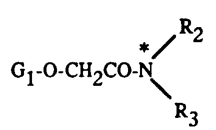 dans laquelle G₁ est défini comme précédemment et 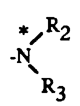 représente le reste d'une base organique optiquement active, puis hydrolyse le produit obtenu de formule générale : 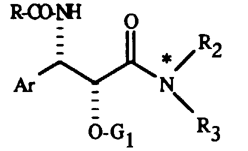 dans laquelle R, Ar, G₁ et 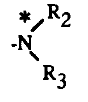 sont définis comme précédemment, et isole le produit obtenu.

2. Procédé selon la revendication 1 caractérisé en ce que R et G₁ étant définis comme dans la revendication 1, Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alkylamino, dialkylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone, que les radicaux alcényles et alcynyles contiennent 3 à 8 atomes de carbone et que les radicaux aryles sont des radicaux phényles ou α- ou β-naphtyles.

3. Procédé selon la revendication 1 caractérisé en ce que R et G₁ étant définis comme dans la revendication 1, Ar représente un radical phényle éventuellement substitué par un ou plusieurs atomes ou radicaux, identiques ou différents, choisis parmi les atomes d'halogène et les radicaux alcoyles, alcoxy, amino, alcoylamino, dialcoylamino, acylamino, alcoxycarbonylamino et trifluorométhyle.

4. Procédé selon l'une des revendications 1 à 3 caractérisé en ce que 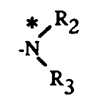 représente un reste L(+)-2,10-camphorsultame de formule

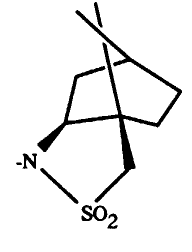

5. Procédé selon l'une des revendications 1 à 4 caractérisé en ce que l'anionisation de l'amide optiquement active de l'acide hydroxyacétique protégé est effectuée au moyen d'un amidure de métal alcalin choisi parmi le bis-(triméthylsilyl) amidure de sodium, le bis-(triméthylsilyl) amidure de lithium, le bis-(triméthylsilyl) amidure de potassium, le diisopropylamidure de lithium, le diéthylamidure de lithium, le dicyclohexylamidure de lithium, (CH₃)₃SiN(R')Li avec R' représentant alcoyle, cycloalcoyle, aryle ou le t.butyllithium.

6. Procédé selon la revendication 5 caractérisé en ce que l'amidure de métal alcalin est le bis-(triméthylsilyl) amidure de lithium.

7. Procédé selon l'une des revendications 5 ou 6 caractérisé en ce que l'anionisation est effectuée en opérant dans un solvant organique inerte à une température inférieure à -30°C.

8. Procédé selon la revendication 7 caractérisé en ce que le solvant est choisi parmi les éthers tels que le tétrahydrofuranne.

9. Procédé selon la revendication 7 caractérisé en ce que l'on opère à -78°C.

10. Procédé selon l'une des revendications 1 à 9 caractérisé en ce que l'action de la N-carbonylarylimine éventuellement préparée in situ sur l'anion de l'amide optiquement active de l'acide hydroxyacétique protégé est effectuée dans un solvant organique inerte à une température inférieure à 0°C.

11. Procédé selon la revendication 10 caractérisé en ce que le solvant organique est choisi parmi les éthers tels que le tétrahydrofuranne.

12. Procédé selon la revendication 10 caractérisé en ce que l'on opère à -78°C.

13. Procédé selon l'une des revendications 1 à 4 caractérisé en ce que l'hydrolyse du produit de condensation de formule générale : 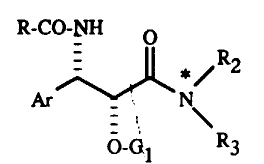 dans laquelle R, Ar, G₁ et 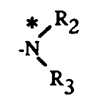 sont définis comme dans les revendications 1 à 4 est effectuée au moyen d'une base minérale en milieu aqueux ou hydroorganique.

14. Procédé selon la revendication 13 caractérisé en ce que l'on opère en outre en présence d'eau oxygénée.

15. Procédé selon l'une des revendications 13 ou 14 caractérisé en ce que l'on opère à une température comprise entre -10 et 20°C.

16. Procédé selon l'une des revendications 13 à 15 caractérisé en ce que la base est la lithine.

17. Les dérivés de la β-phénylisosérine de formule générale : 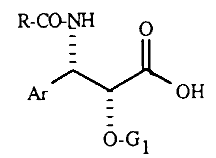 sous forme de sels ou d'esters, dans laquelle R et Ar sont définis comme dans l'une des revendications 1 à 3 et G₁ représente un radical -CH₂-Ph dans lequel Ph représente un radical phényle éventuellement substitué par un ou plusieurs atomes ou radicaux, identiques ou différents, choisis parmi les atomes d'halogène et les radicaux alcoyles contenant 1 à 4 atomes de carbone ou alcoxy contenant 1 à 4 atomes de carbone.

18. La N-carbonylarylimine de formule générale :
Ar-CH=N-CO-R
dans laquelle Ar est défini comme dans l'une des revendications 1 ou 2 et R représente un radical t.butoxy.

19. Utilisation d'un produit selon la revendication 17 pour la préparation d'un dérivé du taxane de formule générale : 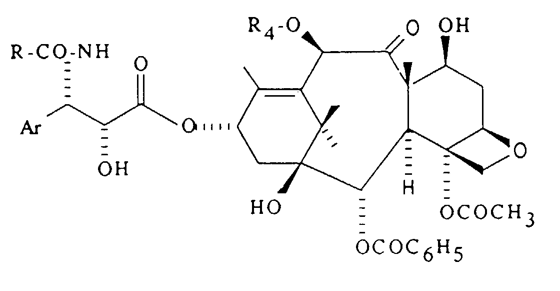 dans laquelle R et Ar sont définis comme dans l'une des revendications 1 à 3 et R₄ représente un atome d'hydrogène ou un radical acétyle caractérisé en ce que l'on fait agir un produit selon la revendication 17 sur un dérivé de la baccatine III ou de la désacétyl-10 baccatine III de formule générale : 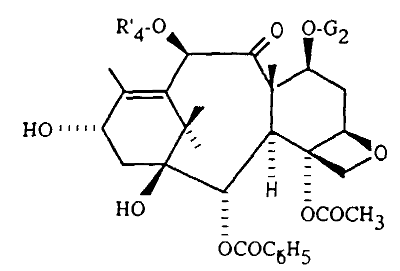 dans laquelle G₂ représente un groupement protecteur de la fonction hydroxy tel que le radical trichloro-2,2,2 éthoxycarbonyle ou un radical trialcoylsilyle et R'₄ représente un radical acétyle ou un groupement protecteur de la fonction hydroxy tel qu'un radical trichloro-2,2,2 éthoxycarbonyle, pour obtenir un produit de formule générale : 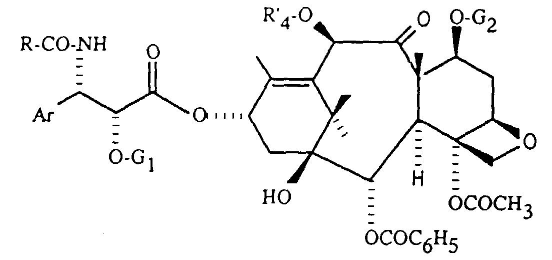 dans laquelle Ar, R et G₁ sont définis comme dans la revendication 17 et G₂ et R'₄ sont définis comme ci-dessus, remplace les groupements protecteurs G₂ et éventuellement R'₄ par des atomes d'hydrogène pour obtenir un produit de formule générale : 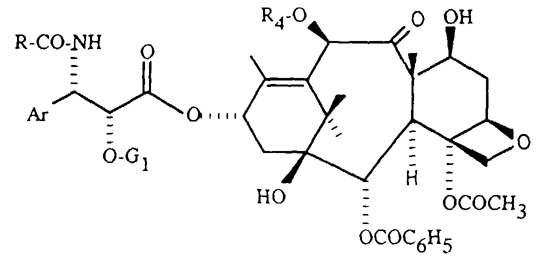 dont on remplace le groupement protecteur G₁ par un atome d'hydrogène et isole le produit obtenu.

20. Utilisation selon la revendication 19 caractérisée en ce que l'estérification est effectuée en présence d'un agent de condensation tel qu'un carbodiimide ou un carbonate réactif et d'un agent d'activation tel qu'une aminopyridine dans un solvant organique choisi parmi les hydrocarbures aromatiques, les éthers, les nitriles et les esters à une température comprise entre 0 et 90°C.

21. Utilisation selon la revendication 19 caractérisée en ce que le remplacement des groupements protecteurs G₂ et éventuellement R'₄ par des atomes d'hydrogène est effectuée par le zinc, éventuellement associé au cuivre, en présence d'acide acétique ou d'un acide minéral ou organique en solution dans un alcool aliphatique lorsque G₂ et/ou R'₄ représentent un radical trichloro-2,2,2 éthoxycarbonyle, ou par traitement en milieu acide lorsque l'un des groupements protecteurs représente un radical silylé.

22. Utilisation selon la revendication 19 caractérisée en ce que le remplacement du groupement protecteur G₁ par un atome d'hydrogène est effectué par hydrogénolyse au moyen d'hydrogène en présence d'un catalyseur tel que le palladium noir ou par l'action de la dichlorodicyanobenzoquinone (DDQ) dans un solvant organique tel que le dichlorométhane ou l'acétonitrile.

23. Les dérivés du taxane de formule générale : 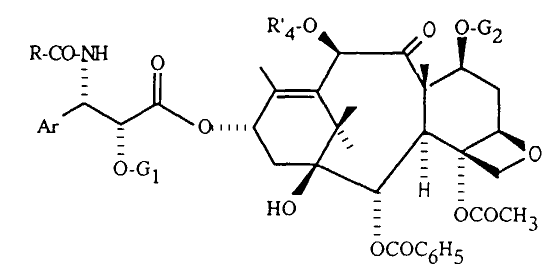 dans laquelle R, Ar et G₁ sont définis comme dans la revendication 17 et G₂ et R'₄ sont définis comme dans la revendication 19.

24. Les dérivés du taxane de formule générale : 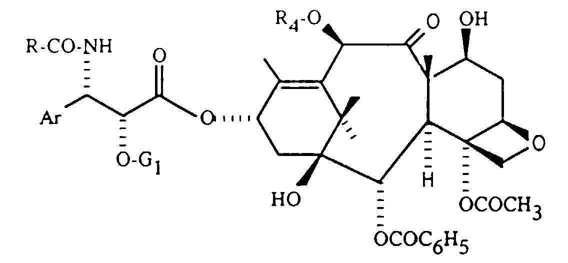 dans laquelle R, Ar et G₁ sont définis comme dans la revendication 17 et R₄ représente un atome d'hydrogène ou le radical acétyle.

## Claims

1. Process for the stereoselective preparation of a β-phenylisoserine derivative of general formula: 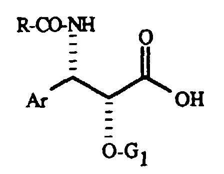 where appropriate in the form of a salt or ester, in which
Ar represents an aryl radical,
R represents a phenyl or α- or β-naphthyl radical optionally substituted with one or more identical or different atoms or radicals chosen from halogen atoms, alkyl radicals containing 1 to 4 carbon atoms and alkoxy radicals containing 1 to 4 carbon atoms, or a radical R₁-O in which R₁ represents:
- an unbranched or branched alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 11 carbon atoms, these radicals being optionally substituted with one or more substituents chosen from halogen atoms and hydroxyl radicals, alkyloxy radicals containing 1 to 4 carbon atoms, dialkylamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl, cyano or carboxyl radicals or alkyloxycarbonyl radicals in which the alkyl portion contains 1 to 4 carbon atoms,
- or a phenyl radical optionally substituted with one or more atoms or radicals chosen from halogen atoms and alkyl radicals containing 1 to 4 carbon atoms or alkyloxy radicals containing 1 to 4 carbon atoms,
- or a saturated or unsaturated 4- or 6-membered nitrogenous heterocyclic radical optionally substituted with one or more alkyl radicals containing 1 to 4 carbon atoms,
on the understanding that the cycloalkyl, cycloalkenyl or bicycloalkyl radicals can be optionally substituted with one or more alkyl radicals containing 1 to 4 carbon atoms, and
G₁ represents a group protecting the hydroxyl function, chosen from methoxymethyl, 1-ethoxyethyl, benzyloxymethyl, 2,2,2-trichloroethoxymethyl, tetrahydrofuryl, tetrahydropyranyl and β-(trimethylsilyl)ethoxymethyl radicals, trialkylsilyl radicals in which the alkyl radicals contain 1 to 4 carbon atoms, or -CH₂-Ph in which Ph represents a phenyl radical optionally substituted with one or more identical or different atoms or radicals chosen from halogen atoms, alkyl radicals containing 1 to 4 carbon atoms and alkoxy radicals containing 1 to 4 carbon atoms,
characterized in that an N-carbonylarylimine of general formula:
Ar-CH=N-CO-R
in which Ar and R are defined as above, is reacted with a previously anionized optically active amide of a protected hydroxyacetic acid, of general formula: 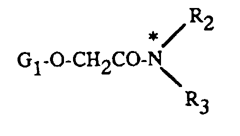 in which G₁ is defined as above and 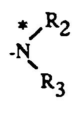 represents the residue of an optically active organic base, the product obtained, of general formula: 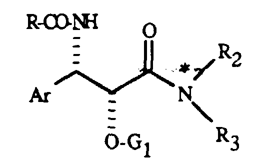 in which R, Ar, G₁ and 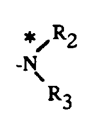 are defined as above, is then hydrolysed, and the product obtained is isolated.

2. Process according to claim 1, characterized in that, R and G₁ being defined as in claim 1, Ar represents a phenyl or α- or β-naphthyl radical optionally substituted with one or more atoms or radicals chosen from halogen (fluorine, chlorine, bromine, iodine) atoms and alkyl, alkenyl, alkynyl, aryl, arylalkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, dialkylcarbamoyl, cyano, nitro and trifluoromethyl radicals, on the understanding that the alkyl radicals and alkyl portions of the other radicals contain 1 to 4 carbon atoms, that the alkenyl and alkynyl radicals contain 3 to 8 carbon atoms and that the aryl radicals are phenyl or *α-* or β-naphthyl radicals.

3. Process according to claim 1, characterized in that, R and G₁ being defined as in claim 1, Ar represents a phenyl radical optionally substituted with one or more identical or different atoms or radicals chosen from halogen atoms and alkyl, alkoxy, amino, alkylamino, dialkylamino, acylamino, alkoxycarbonylamino and trifluoromethyl radicals.

4. Process according to one of claims 1 to 3, characterized in that 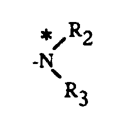 represents an L(+) -2,10-camphorsultam residue of formula:

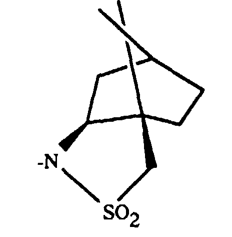

5. Process according to one of claims 1 to 4, characterized in that the anionization of the optically active amide of the protected hydroxyacetic acid is effected by means of an alkali metal amide chosen from sodium bis (trimethylsilyl) amide, lithium bis (trimethylsilyl) amide, potassium bis(trimethyl-silyl)amide, lithium diisopropylamide, lithium diethylamide, lithium dicyclohexylamide and (CH₃)₃SiN (R') Li with R' representing alkyl, cycloalkyl or aryl, or t-butyllithium.

6. Process according to claim 5, characterized in that the alkali metal amide is lithium bis(trimethylsilyl) amide.

7. Process according to one of claims 5 and 6, characterized in that the anionization is performed working in an inert organic solvent at a temperature below -30°C.

8. Process according to claim 7, characterized in that the solvent is chosen from ethers such as tetrahydrofuran.

9. Process according to claim 7, characterized in that the anionization is performed at -78°C.

10. Process according to one of claims 1 to 9, characterized in that the action of the N-carbonylarylimine, optionally prepared in situ, on the anion of the optically active amide of the protected hydroxyacetic acid is performed in an inert organic solvent at a temperature below 0°C.

11. Process according to claim 10, characterized in that the organic solvent is chosen from ethers such as tetrahydrofuran.

12. Process according to claim 10, characterized in that the reaction is performed at -78°C.

13. Process according to one of claims 1 to 4, characterized in that the hydrolysis of the condensation product of general formula: 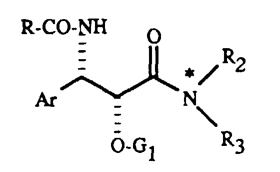 in which R, Ar, G₁ and 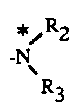 are defined as in claims 1 to 4, is performed by means of an inorganic base in an aqueous or aqueous-organic medium.

14. Process according to claim 13, characterized in that the hydrolysis is performed, in addition, in the presence of hydrogen peroxide.

15. Process according to one of claims 13 and 14, characterized in that the hydrolysis is performed at a temperature of between -10 and 20°C.

16. Process according to one of claims 13 to 15, characterized in that the base is lithium hydroxide.

17. The β-phenylisoserine derivatives of general formula: 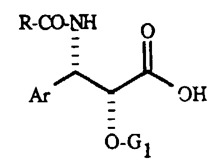 in the form of salts or esters, in which R and Ar are defined as in one of claims 1 to 3 and G₁ represents a -CH₂-Ph radical in which Ph represents a phenyl radical optionally substituted with one or more identical or different atoms or radicals chosen from halogen atoms and alkyl radicals containing 1 to 4 carbon atoms or alkoxy radicals containing 1 to 4 carbon atoms.

18. The N-carbonylarylimine of general formula:
Ar-CH=N-CO-R
in which Ar is defined as in one of claims 1 and 2 and R represents a t-butoxy radical.

19. Use of a product according to claim 17, for the preparation of a taxane derivatives of general formula: 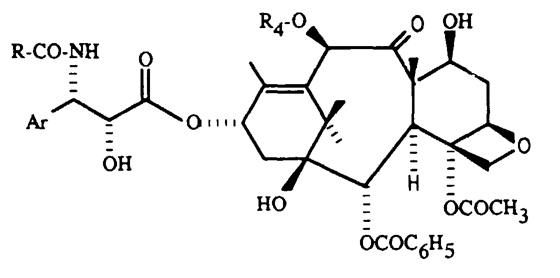 in which R and Ar are defined as in one of claims 1 to 3 and R₄ represents a hydrogen atom or an acetyl radical, characterized in that a product according to claim 17 is reacted with a baccatin III or 10-deacetylbaccatin III derivative of general formula: 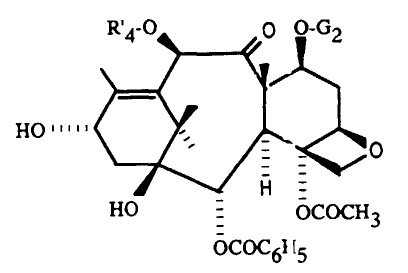 in which G₂ represents a group protecting the hydroxyl function, such as a 2,2,2-trichloroethoxycarbonyl radical or a trialkylsilyl radical, and R'₄ represents an acetyl radical or a group protecting the hydroxyl function, such as a 2,2,2-trichloroethoxycarbonyl radical, to obtain a product of general formula: 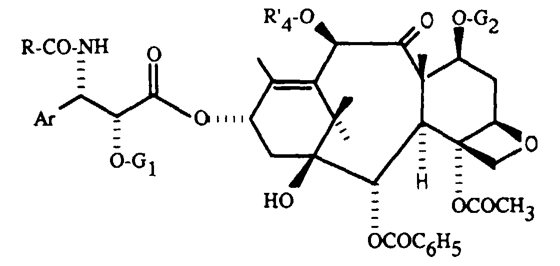 in which Ar, R and G₁ are defined as in claim 17 and G₂ and R'₄ are defined as above, the protective groups G₂ and, where appropriate, R'₄ are replaced by hydrogen atoms to obtain a product of general formula: 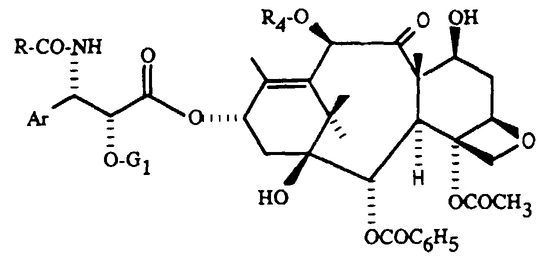 the protective group G₁ of which is replaced by a hydrogen atom, and the product obtained is isolated.

20. Use according to claim 19, characterized in that the esterification is performed in the presence of a condensing agent such as a carbodiimide or a reactive carbonate and an activating agent such as an aminopyridine, in an organic solvent chosen from aromatic hydrocarbons, ethers, nitriles and esters, at a temperature of between 0 and 90°C.

21. Use according to claim 19, characterized in that the replacement of the protective groups G₂ and, where appropriate, R'₄ by hydrogen atoms is performed with zinc, optionally in combination with copper, in the presence of acetic acid or an inorganic or organic acid dissolved in an aliphatic alcohol when G₂ and/or R'₄ represent a 2,2,2-trichloroethoxycarbonyl radical, or by treatment in an acid medium when one of the protective groups represents a silyl radical.

22. Use according to claim 19, characterized in that the replacement of the protective group G₁ by a hydrogen atom is performed by hydrogenolysis by means of hydrogen in the presence of a catalyst such as palladium black, or by the action of dichlorodicyanobenzoquinone (DDQ) in an organic solvent such as dichloromethane or acetonitrile.

23. The taxane derivatives of general formula: 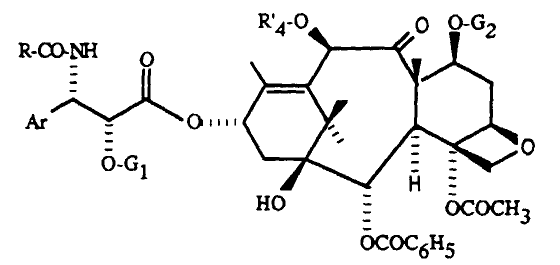 in which R, Ar and G₁ are defined as in claim 17 and G₂ and R'₄ are defined as in claim 19.

24. The taxane derivatives of general formula: 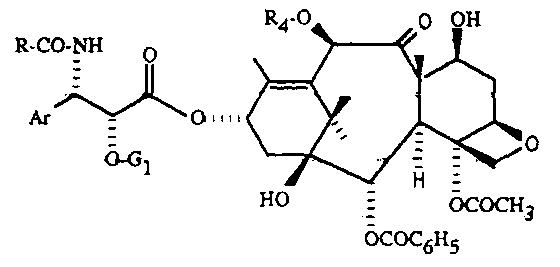 in which R, Ar and G₁ are defined as in claim 17 and R₄ represents a hydrogen atom or an acetyl radical.

## Patentansprüche

1. Verfahren zur stereoselektiven Herstellung eines ß-Phenylisoserin-Derivates der allgemeinen Formel 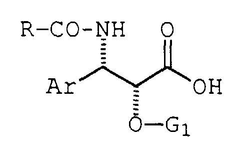 gegebenenfalls in Form von Salz oder Ester, in der
Ar einen Arylrest darstellt,
R einen Rest Phenyl oder α- oder ß-Naphthyl darstellt, gegebenenfalls substituiert durch ein oder mehrere gleiche oder verschiedene Atome oder Reste, ausgewählt unter den Halogenatomen und den Resten Alkyl mit 1 bis 4 Kohlenstoffatomen, Alkoxy mit 1 bis 4 Kohlenstoffatomen oder einem Rest R₁-O, worin R₁ ist:
- ein gerader oder verzweigter Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen oder Bicycloalkyl mit 7 bis 11 Kohlenstoffatomen, wobei diese Reste gegebenenfalls substituiert sind durch einen oder mehrere Substituenten, ausgewählt unter den Halogenatomen und den Resten Hydroxy, Alkyloxy mit 1 bis 4 Kohlenstoffatomen, Dialkylamino, von dem jeder Alkylteil 1 bis 4 Kohlenstoffatome enthält, Piperidino, Morpholino, 1Piperazinyl (gegebenenfalls substituiert in -4 durch einen Alkylrest mit 1 bis 4 Kohlenstoffatomen oder durch einen Phenylalkylrest, dessen Alkylteil 1 bis 4 Kohlenstoffatome enthält), Cycloalkyl mit 3 bis 6 Kohlenstoffatomen, Cycloalkenyl mit 4 bis 6 Kohlenstoffatomen, Phenyl, Cyano, Carboxy oder Alkyloxycarbonyl, dessen Alkylteil 1 bis 4 Kohlenstoffatome enthält,
- oder ein Rest Phenyl, gegebenenfalls substituiert durch ein oder mehrere Atome oder Reste, ausgewählt unter den Halogenatomen und den Resten Alkyl mit 1 bis 4 Kohlenstoffatomen oder Alkyloxy mit 1 bis 4 Kohlenstoffatomen,
- oder ein Rest eines gesättigten oder ungesättigten Stickstoff-Heterocyclus mit 4 oder 6 Ringgliedern und gegebenenfalls substituiert durch einen oder mehrere Alkylreste mit 1 bis 4 Kohlenstoffatomen,
mit der Maßgabe, daß die Reste Cycloalkyl, Cycloalkenyl oder Bicycloalkyl gegebenenfalls durch einen oder mehrere Alkylreste mit 1 bis 4 Kohlenstoffatomen substituiert sein können, und
G₁ eine Schutzgruppe für die Hydroxyfunktion darstellt, ausgewählt unter den Resten Methoxymethyl, 1-Ethoxy-ethyl, Benzyloxymethyl, 2,2,2-Trichlor-ethoxymethyl, Tetrahydrofuranyl, Tetrahydropyranyl, ß-(Trimethylsilyl)-ethoxymethyl, Trialkylsilyl, dessen Alkylreste 1 bis 4 Kohlenstoffatome enthalten, oder -CH₂-Ph, worin Ph ein Phenylrest ist, gegebenenfalls substituiert durch ein oder mehrere, gleiche oder verschiedene Atome oder Reste, ausgewählt unter den Halogenatomen, den Resten Alkyl mit 1 bis 4 Kohlenstoffatomen und Alkoxy mit 1 bis 4 Kohlenstoffatomen,
dadurch gekennzeichnet, daß man ein N-Carbonylarylimin der allgemeinen Formel
Ar-CH=N-CO-R
in der Ar und R wie oben definiert sind, mit einem optisch aktiven Amid einer geschützten, zuvor anionisierten Hydroxyessigsäure der allgemeinen Formel 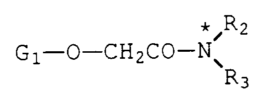 zur Reaktion bringt, in der G₁ wie oben definiert ist und 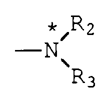 den Rest einer optisch aktiven organischen Base darstellt, wonach man das erhaltene Produkt der allgemeinen Formel 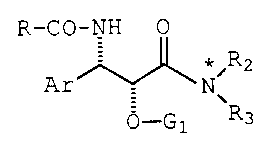 in der R, Ar, G₁ und 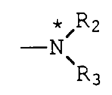 wie oben definiert sind, hydrolysiert und das erhaltene Produkt isoliert.

2. Verfahren nach Anspruch 1, dadurch gekennzeichnet, daß R und G₁ wie in Anspruch 1 definiert sind, Ar einen Rest Phenyl oder αoder ß-Naphthyl darstellt, gegebenenfalls substituiert durch ein oder mehrere Atome oder Reste, ausgewählt unter den Halogenatomen (Fluor, Chlor, Brom, Iod) und den Resten Alkyl, Alkenyl, Alkinyl, Aryl, Arylalkyl, Alkoxy, Alkylthio, Aryloxy, Arylthio, Hydroxy, Hydroxyalkyl, Mercapto, Formyl, Acyl, Acylamino, Arylamino, Alkoxycarbonylamino, Amino, Alkylamino, Dialkylamino, Carboxy, Alkoxycarbonyl, Carbamoyl, Dialkylcarbamoyl, Cyano, Nitro und Trifluormethyl, mit der Maßgabe, daß die Alkylreste und Alkylteile der anderen Reste 1 bis 4 Kohlenstoffatome, die Reste Alkenyl und Alkinyl 3 bis 8 Kohlenstoffatome enthalten und die Arylreste Phenylreste oder α- oder ß-Naphthylreste sind.

3. Verfahren nach Anspruch 1, dadurch gekennzeichnet, daß R und G₁ wie in Anspruch 1 definiert sind und Ar einen Phenylrest darstellt, gegebenenfalls substituiert durch ein oder mehrere, gleiche oder verschiedene Atome oder Reste, ausgewählt unter den Halogenatomen und den Resten Alkyl, Alkoxy, Amino, Alkylamino, Dialkylamino, Acylamino, Alkoxycarbonylamino und Trifluormethyl.

4. Verfahren nach einem der Ansprüche 1 bis 3, dadurch gekennzeichnet, daß 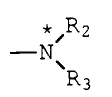 einen Rest L(+)-2,10-Camphorsultam der Formel 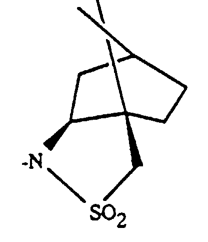 darstellt.

5. Verfahren nach einem der Ansprüche 1 bis 4, dadurch gekennzeichnet, daß die Anionisierung des optisch aktiven Amids der geschützten Hydroxyessigsäure mit Hilfe eines Alkalimetallamids durchgeführt wird, ausgewählt unter Natrium-bis-(trimethylsilyl)-amid, Lithium-bis-(trimethylsilyl)-amid, Kalium-bis-(trimethylsilyl)-amid, Lithium-diisopropylamid, Lithium-diethylamid, Lithium-dicyclohexylamid, (CH₃)₃SiN (R') Li worin R' Alkyl, Cycloalkyl, Aryl darstellt, oder tert.-Butyllithium.

6. Verfahren nach Anspruch 5, dadurch gekennzeichnet, daß das Alkalimetallamid das Lithium-bis-(trimethylsilyl)-amid ist.

7. Verfahren nach einem der Ansprüche 5 oder 6, dadurch gekennzeichnet, daß die Anionisierung in einem inerten organischen Lösungsmittel bei einer Temperatur von unter -30 °C durchgeführt wird.

8. Verfahren nach Anspruch 7, dadurch gekennzeichnet, daß das Lösungsmittel unter den Ethern wie Tetrahydrofuran ausgewählt wird.

9. Verfahren nach Anspruch 7, dadurch gekennzeichnet, daß man bei -78 °C arbeitet.

10. Verfahren nach einem der Ansprüche 1 bis 9, dadurch gekennzeichnet, daß die Einwirkung des gegebenenfalls in situ hergestellten N-Carbonylarylimins auf das Anion des optisch aktiven Amids der geschützten Hydroxyessigsäure in einem inerten organischen Lösungsmittel bei einer Temperatur von unterhalb 0 °C durchgeführt wird.

11. Verfahren nach Anspruch 10, dadurch gekennzeichnet, daß das organische Lösungsmittel unter den Ethern wie Tetrahydrofuran ausgewählt wird.

12. Verfahren nach Anspruch 10, dadurch gekennzeichnet, daß man bei -78 °C arbeitet.

13. Verfahren nach einem der Ansprüche 1 bis 4, dadurch gekennzeichnet, daß die Hydrolyse des Kondensationsproduktes der allgemeinen Formel 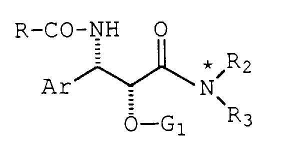 in der R, Ar, G₁ und 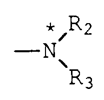 wie in den Ansprüchen 1 bis 4 definiert sind, mit Hilfe einer Mineralbase im wäßrigen oder wäßrig-organischen Medium durchgeführt wird.

14. Verfahren nach Anspruch 13, dadurch gekennzeichnet, daß man außerdem in Anwesenheit von Wasserstoffperoxid arbeitet.

15. Verfahren nach einem der Ansprüche 13 oder 14, dadurch gekennzeichnet, daß man bei einer Temperatur zwischen -10 °C und 20 °C arbeitet.

16. Verfahren nach einem der Ansprüche 13 bis 15, dadurch gekennzeichnet, daß die Base Lithiumhydroxid ist.

17. Die β-Phenylisoserin-Derivate der allgemeinen Formel 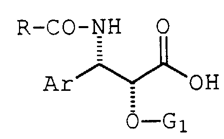 in Form von Salzen oder Estern, in der R und Ar wie in einem der Ansprüche 1 bis 3 definiert sind und G₁ einen Rest -CH₂-Ph darstellt, worin Ph ein Phenylrest ist, gegebenenfalls substituiert durch ein oder mehrere, gleiche oder verschiedene Atome oder Reste, ausgewählt unter den Halogenatomen und den Resten Alkyl mit 1 bis 4 Kohlenstoffatomen oder Alkoxy mit 1 bis 4 Kohlenstoffatomen.

18. Das N-Carbonylarylimin der allgemeinen Formel
Ar-CH=N-CO-R
in der Ar wie in einem der Ansprüche 1 oder 2 definiert ist und R einen Rest tert.-Butoxy bedeutet.

19. Verwendung eines Produktes gemäß Anspruch 17 zur Herstellung eines Taxan-Derivates der allgemeinen Formel 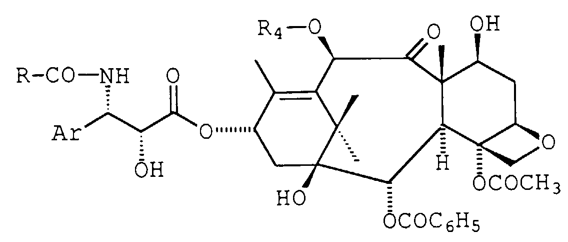 in der R und Ar wie in einem der Ansprüche 1 bis 3 definiert sind und R₄ ein Wasserstoffatom oder einen Acetylrest darstellt, dadurch gekennzeichnet, daß man ein Produkt nach Anspruch 17 mit einem Derivat von Baccatin III oder 10-Desacetyl-Baccatin III der allgemeinen Formel 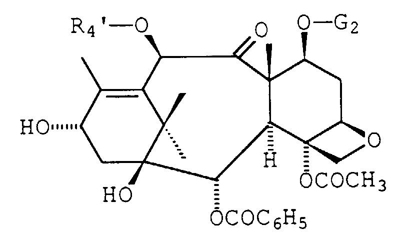 zur Reaktion bringt, in der G₂ eine Schutzgruppe für die Hydroxyfunktion darstellt, wie der Rest 2,2,2-Trichlor-ethoxycarbonyl oder ein Rest Trialkylsilyl und R₄' einen Acetylrest oder eine Schutzgruppe für die Hydroxyfunktion bedeutet, wie einen Rest 2,2,2-Trichlor-ethoxycarbonyl, um ein Produkt der allgemeinen Formel 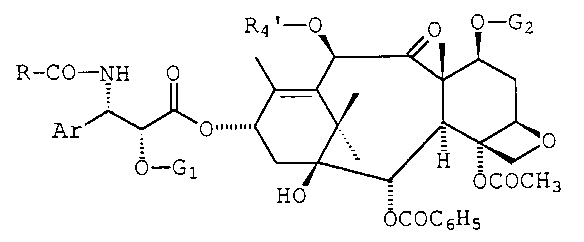 zu erhalten, in der Ar, R und G₁ wie in Anspruch 17 und G₂ und R₄' wie oben definiert sind, und man die Schutzgruppen G₂ und gegebenenfalls R₄' durch Wasserstoffatome austauscht, um ein Produkt der allgemeinen Formel 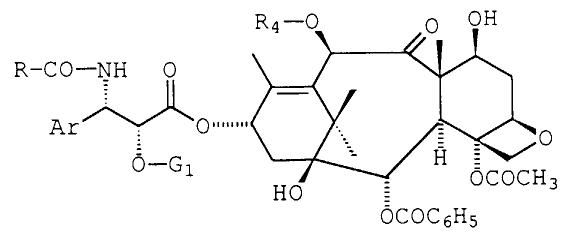 zu erhalten, bei dem man dann die Schutzgruppe G₁ durch ein Wasserstoffatom ersetzt und das erhaltene Produkt isoliert.

20. Verwendung nach Anspruch 19, dadurch gekennzeichnet, daß die Veresterung in Anwesenheit eines Kondensationsmittels wie einem Carbodiimid oder einem reaktiven Carbonat und eines Aktivierungsmittels wie einem Aminopyridin in einem organischen Lösungsmittel, ausgewählt unter den aromatischen Kohlenwasserstoffen, den Ethern, den Nitrilen und den Estern, bei einer Temperatur zwischen 0 °C und 90 °C durchgeführt wird.

21. Verwendung nach Anspruch 19, dadurch gekennzeichnet, daß der Austausch der Schutzgruppen G₂ und gegebenenfalls R₄' durch Wasserstoffatome mit Hilfe einer Behandlung durch Zink, gegebenenfalls assoziiert mit Kupfer, in Anwesenheit von Essigsäure oder einer Mineralsäure oder organischen Säure in Lösung eines aliphatischen Alkohols durchgeführt wird, wenn G₂ und/oder R₄' einen Rest 2,2,2-Trichlor-ethoxycarbonyl darstellen, oder durch Behandlung im sauren Medium, wenn eine der Schutzgruppen ein Silylrest ist.

22. Verwendung nach Anspruch 19, dadurch gekennzeichnet, daß der Austausch der Schutzgruppe G₁ durch ein Wasserstoffatom mittels Hydrogenolyse mit Wasserstoff in Anwesenheit eines Katalysators wie Palladium-Kohle oder durch Einwirkung von Dichlordicyanobenzochinon (DDQ) in einem organischen Lösungsmittel wie Dichlormethan oder Acetonitril durchgeführt wird.

23. Die Taxan-Derivate der allgemeinen Formel 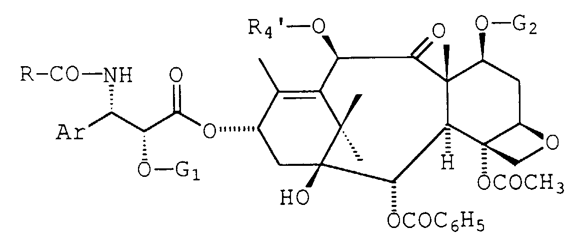 in der R, Ar und G₁ wie in Anspruch 17 und G₂ und R₄' wie in Anspruch 19 definiert sind.

24. Die Taxan-Derivate der allgemeinen Formel 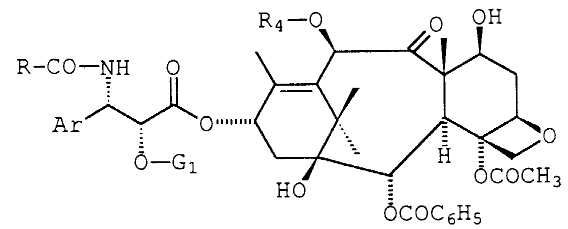 in der R, Ar und G₁ wie in Anspruch 17 definiert sind und R₄ ein Wasserstoffatom oder den Acetylrest darstellt.
